# FASCICULE DE BREVET EUROPEEN

(11) **EP 4 598 613 B1**
(45) Date de publication et mention de la délivrance du brevet: **01.07.2026**
(21) Numéro de dépôt: 23776386.7
(22) Date de dépôt: 25.09.2023
(51) Int. Cl.: A61M 5/315

(54) **SERINGUE MANUELLE AVEC POUSSOIR FLEXIBLE**
MANUELLE SPRITZE MIT FLEXIBLEM KOLBEN
MANUAL SYRINGE HAVING FLEXIBLE PLUNGER

(30) Priorité: 06.10.2022 FR 2210240
(43) Date de publication de la demande: 13.08.2025
(73) Titulaire: Laboratoires Vivacy, 75116 Paris (FR)
(72) Inventeur: ROCHEDIX, Emma, 74160 Saint Julien en Genevois (FR); SUC, Pierre, 74000 Annecy (FR); MOULDI, Safia, 01630 Saint-Genis-Pouilly (FR)
(74) Mandataire: INNOV-GROUP
(86) Numéro de dépôt international: PCT/EP2023/076395
(87) Numéro de publication internationale: WO 2024/074329

(56) Documents cités:
- GB-A- 2 603 181
- US-A1- 2017 290 987
- US-A1- 2020 078 528

## Description

### Domaine technique

L'invention se rapporte au domaine technique des seringues manuelles, c'est-à-dire des seringues comportant un poussoir sur lequel est exercée manuellement une pression d'injection par un professionnel de santé.

L'invention trouve notamment son application dans l'injection de gels viscoélastiques (e.g. à base d'acide hyaluronique), par exemple dans le domaine de l'anti-âge et de l'esthétique médicale.

### État de l'art

Une seringue manuelle connue de l'état de la technique comporte :
- un corps de seringue, destiné à recevoir un produit à injecter ; le corps de seringue présentant une première extrémité, destinée à être équipée d'un porte-aiguille, et une seconde extrémité opposée ;
- des ailettes de préhension, agencées à la seconde extrémité du corps de seringue ;
- une tige de piston, montée coulissante à l'intérieur du corps de seringue, la tige de piston présentant des première et seconde extrémités opposées ; la tige de piston possédant une longueur ;
- un butoir, agencé à la première extrémité de la tige de piston ;
- un poussoir manuel, agencé à la seconde extrémité de la tige de piston, et sur lequel est exercée manuellement une pression d'injection adaptée pour injecter le produit.

Une telle seringue manuelle de l'état de la technique n'est pas entièrement satisfaisante dans le cas où le produit à injecter nécessite une pression importante à exercer sur le poussoir. C'est notamment le cas lorsque le produit à injecter est un gel viscoélastique à base d'acide hyaluronique, où il est parfois nécessaire d'appliquer une force supérieure à 50 N. Le professionnel de santé (e.g. médecin) est donc susceptible de ressentir un certain inconfort, voire des douleurs, en utilisant de manière répétée une telle seringue de l'état de la technique. A volume constant, la seringue manuelle est généralement conçue pour allonger et affiner le corps de seringue et la tige de piston afin de réduire la surface de contact avec le produit à injecter, et par là-même réduire la force de viscosité. Toutefois, l'allongement et l'affinement de la tige de piston conduisent également à un inconfort pour le professionnel de santé. Cet inconfort pour la préhension de la seringue est particulièrement manifeste en début d'injection, lorsque la seconde extrémité de la tige de piston est située à une distance importante des ailettes de préhension.

De plus, le produit peut être injecté au niveau des rides d'un patient, plus particulièrement sous les yeux. Une telle injection requiert donc une excellente stabilité afin d'obtenir la précision souhaitée (pouvant être de l'ordre de ± 1 mm) et atteindre les rides du patient de manière effective et sécurisée. Comme évoqué précédemment, l'allongement et l'affinement de la tige de piston conduisent à un inconfort pour la préhension de la seringue, susceptible de se traduire par un manque de stabilité et une perte de précision, tout particulièrement en début d'injection.

### Exposé de l'invention

L'invention vise à remédier en tout ou partie aux inconvénients précités. A cet effet, l'invention a pour objet une seringue manuelle, comportant :
- un corps de seringue, destiné à recevoir un produit à injecter ; le corps de seringue présentant une première extrémité, destinée à être équipée d'un porte-aiguille, et une seconde extrémité opposée ;
- des ailettes de préhension, agencées à la seconde extrémité du corps de seringue ;
- une tige de piston, montée coulissante à l'intérieur du corps de seringue, la tige de piston présentant des première et seconde extrémités opposées ; la tige de piston possédant une longueur ;
- un butoir, agencé à la première extrémité de la tige de piston ;
- un poussoir manuel, agencé à la seconde extrémité de la tige de piston, et sur lequel est exercée manuellement une pression d'injection adaptée pour injecter le produit ;
remarquable en ce que le poussoir manuel comprend :
- une partie rigide ;
- une partie déformable, conçue pour se fléchir vers la tige de piston selon une flèche comprise entre 5% et 20% de la longueur de la tige de piston, lorsque la pression d'injection est exercée manuellement sur le poussoir manuel.

La partie déformable peut être conçue pour se fléchir vers la tige de piston selon une flèche comprise entre 5% et 25% de la longueur de la tige de piston, lorsque la pression d'injection est exercée manuellement sur le poussoir manuel. La flèche est avantageusement comprise entre 10% et 25%, plus préférentiellement comprise entre 15% et 25% de la longueur de la tige de piston, lorsque la pression d'injection est exercée manuellement sur le poussoir manuel.

Ainsi, une seringue manuelle selon l'invention permet, grâce à une telle partie déformable, d'améliorer le confort du professionnel de santé :
(i) durant l'injection en épousant la forme de la paume de la main du professionnel de santé, et
(ii) tout particulièrement en début d'injection en rapprochant la partie déformable du poussoir (et donc la main du professionnel de santé) vers les ailettes de préhension.

Il en résulte une meilleure stabilité de la seringue, autorisant une grande précision lors de l'injection.

La seringue manuelle selon l'invention peut comporter une ou plusieurs des caractéristiques suivantes.

Selon une caractéristique de l'invention, la partie déformable est réalisée dans un premier matériau possédant un premier module d'Young ; et la partie déformable comporte des zones d'affaiblissement du premier matériau, agencées en fonction du premier module d'Young pour fléchir le premier matériau vers la tige de piston selon la flèche comprise entre 5% et 20% de la longueur de la tige de piston, lorsque la pression d'injection est exercée manuellement sur le poussoir manuel.

Les zones d'affaiblissement du premier matériau peuvent être agencées en fonction du premier module d'Young pour fléchir le premier matériau vers la tige de piston selon une flèche comprise entre 5% et 25% (préférentiellement entre 10% et 25%, plus préférentiellement entre 15% et 25%) de la longueur de la tige de piston, lorsque la pression d'injection est exercée manuellement sur le poussoir manuel.

Ainsi, un avantage procuré par les zones d'affaiblissement est d'autoriser une certaine liberté dans le choix du premier matériau. Le premier matériau peut être un matériau rigide ou un matériau souple. L'agencement des zones d'affaiblissement (leur nombre, leur répartition spatiale, leur volume etc.) est ajusté en fonction du premier module d'Young afin de fléchir le premier matériau vers la tige de piston selon la flèche souhaitée.

Selon une caractéristique de l'invention, chaque zone d'affaiblissement du premier matériau forme un évidement de matière, l'évidement de matière formant de préférence une rainure au sein du premier matériau.

Ainsi, un avantage procuré est la facilité de fabrication de telles zones d'affaiblissement, par exemple par moulage.

Selon une caractéristique de l'invention, les zones d'affaiblissement du premier matériau sont agencées pour former un réseau de motifs périodiques.

Ainsi, un avantage procuré est d'optimiser l'étendue spatiale des zones d'affaiblissement pour obtenir la flèche souhaitée.

Selon une caractéristique de l'invention :
- la partie déformable du poussoir manuel est mobile entre une position de repos et une position fléchie dans laquelle la partie déformable est fléchie vers la tige de piston selon la flèche comprise entre 5% et 20% de la longueur de la tige de piston ;
- les zones d'affaiblissement s'étendent suivant des axes parallèles entre eux lorsque la partie déformable est dans la position de repos.

La partie déformable du poussoir manuel peut être mobile entre une position de repos et une position fléchie dans laquelle la partie déformable est fléchie vers la tige de piston selon une flèche comprise entre 5% et 25% (préférentiellement entre 10% et 25%, plus préférentiellement entre 15% et 25%) de la longueur de la tige de piston.

Ainsi, un avantage procuré est la facilité de fabrication de telles zones d'affaiblissement, par exemple par moulage.

Selon une caractéristique de l'invention, le premier matériau est rigide, et la partie rigide du poussoir manuel est réalisée dans le premier matériau.

Ainsi, un avantage procuré est de pouvoir réaliser la partie rigide et la partie déformable du poussoir à partir du même matériau, ce qui permet de diminuer le temps d'opération pour fabriquer la seringue.

Selon une caractéristique de l'invention, le premier matériau est souple.

Ainsi, un avantage procuré est d'autoriser une plus grande liberté pour l'agencement des zones d'affaiblissement que lorsque le premier matériau est rigide afin d'obtenir la flèche souhaitée.

Selon une caractéristique de l'invention, la partie déformable du poussoir manuel est réalisée dans un matériau souple possédant un module d'Young adapté pour fléchir le matériau souple vers la tige de piston selon la flèche comprise entre 5% et 20% de la longueur de la tige de piston, lorsque la pression d'injection est exercée manuellement sur le poussoir manuel.

La partie déformable du poussoir manuel peut être réalisée dans un matériau souple possédant un module d'Young adapté pour fléchir le matériau souple vers la tige de piston selon une flèche comprise entre 5% et 25% (préférentiellement entre 10% et 25%, plus préférentiellement entre 15% et 25%) de la longueur de la tige de piston, lorsque la pression d'injection est exercée manuellement sur le poussoir manuel.

Ainsi, un avantage procuré est de s'affranchir de la réalisation de zones d'affaiblissement. En d'autres termes, la partie déformable du poussoir manuel est dépourvue de zones d'affaiblissement du matériau souple.

Selon une caractéristique de l'invention :
- la tige de piston s'étend suivant un premier axe longitudinal ;
- le poussoir manuel s'étend suivant un deuxième axe longitudinal, perpendiculaire au premier axe longitudinal.

Selon une caractéristique de l'invention :
- la partie déformable du poussoir manuel est mobile entre une position de repos et une position fléchie dans laquelle la partie déformable est fléchie vers la tige de piston selon la flèche comprise entre 5% et 20% de la longueur de la tige de piston ;
- les zones d'affaiblissement du premier matériau s'étendent chacune suivant un axe perpendiculaire aux premier et deuxième axes longitudinaux lorsque la partie déformable est dans la position de repos.

La partie déformable du poussoir manuel peut être mobile entre une position de repos et une position fléchie dans laquelle la partie déformable est fléchie vers la tige de piston selon une flèche comprise entre 5% et 25% (préférentiellement entre 10% et 25%, plus préférentiellement entre 15% et 25%) de la longueur de la tige de piston.

Ainsi, un avantage procuré est d'optimiser l'étendue spatiale des zones d'affaiblissement pour obtenir la flèche souhaitée.

Selon une caractéristique de l'invention :
- la partie déformable du poussoir manuel est mobile entre une position de repos et une position fléchie dans laquelle la partie déformable est fléchie vers la tige de piston selon la flèche comprise entre 5% et 20% de la longueur de la tige de piston ;
- les zones d'affaiblissement du premier matériau présentent une section longitudinale, suivant le premier axe longitudinal, définissant une forme en U inversé ou en V inversé lorsque la partie déformable est dans la position de repos et lorsque le premier axe longitudinal est un axe vertical, la seconde extrémité de la tige de piston étant située au-dessus de la première extrémité de la tige de piston par rapport à l'axe vertical.

La partie déformable du poussoir manuel peut être mobile entre une position de repos et une position fléchie dans laquelle la partie déformable est fléchie vers la tige de piston selon une flèche comprise entre 5% et 25% (préférentiellement entre 10% et 25%, plus préférentiellement entre 15% et 25%) de la longueur de la tige de piston.

Ainsi, un avantage procuré par de telles formes est d'optimiser l'étendue spatiale des zones d'affaiblissement pour obtenir la flèche souhaitée.

Selon une caractéristique de l'invention :
- le poussoir manuel possède une longueur suivant le deuxième axe longitudinal ;
- les zones d'affaiblissement du premier matériau possèdent une longueur totale, suivant le deuxième axe longitudinal, comprise entre 30% et 50% de la longueur du poussoir manuel, lorsque la partie déformable est dans la position de repos.

Ainsi, un avantage procuré est d'obtenir un bon compromis en termes de répartition spatiale entre la partie rigide du poussoir manuel (nécessaire à une poussée efficace pour l'injection) et la partie déformable du poussoir manuel (nécessaire au confort et à la précision de l'injection).

Selon une caractéristique de l'invention :
- le poussoir manuel comporte une partie supérieure sur laquelle est exercée manuellement une pression d'injection adaptée pour injecter le produit, et une partie inférieure opposée à la partie supérieure ;
- la partie inférieure du poussoir manuel comporte deux organes de butée agencés de part et d'autre du premier axe longitudinal de manière à bloquer un glissement de doigts suivant le deuxième axe longitudinal.

Ainsi, un avantage procuré est d'obtenir une meilleure stabilité des doigts positionnés sous le poussoir manuel, ce qui est particulièrement utile lors d'un test d'aspiration pouvant être exercé par un professionnel de santé. Un test d'aspiration consiste à vérifier que l'aiguille ne se trouve pas dans une veine d'un patient, dans le cadre d'une application de type injection par voie intradermique. Pour ce faire, le professionnel de santé procède à une aspiration en tirant sur le poussoir, suivant le sens opposé au sens d'injection du produit, afin de vérifier l'absence d'un reflux sanguin. Ce test d'aspiration conduit à exercer une force importante sur la partie inférieure du poussoir manuel de sorte que les doigts du professionnel de santé sont susceptibles de glisser suivant le deuxième axe longitudinal. De tels organes de butée créent une obstruction physique aux doigts positionnés sur la partie inférieure du poussoir manuel, et permettent donc de sécuriser le test d'aspiration en s'opposant à un glissement des doigts suivant le deuxième axe longitudinal.

Selon une caractéristique de l'invention :
- le poussoir manuel comporte successivement une première zone latérale, une zone centrale et une seconde zone latérale suivant le deuxième axe longitudinal ;
- la zone centrale forme la partie rigide ;
- la première zone latérale et/ou la seconde zone latérale forment la partie déformable.

Ainsi, un avantage procuré est de pouvoir épouser la forme de la paume de la main du professionnel de santé lors de l'injection.

Selon une caractéristique de l'invention :
- le poussoir manuel comporte une zone centrale et une zone périphérique s'étendant autour de la zone centrale ;
- la zone centrale forme la partie rigide ;
- la zone périphérique forme la partie déformable.

Ainsi, un avantage procuré est de pouvoir épouser la forme de la paume de la main du professionnel de santé lors de l'injection.

Selon une caractéristique de l'invention, le poussoir manuel est constitué d'une tête de piston formée à la seconde extrémité de la tige de piston, la tête de piston comprenant la partie rigide et la partie déformable.

Ainsi, un avantage procuré est de diminuer le temps d'opération pour fabriquer la seringue.

Selon une caractéristique de l'invention, le poussoir manuel comporte :
- une tête de piston, rigide, formée à la seconde extrémité de la tige de piston ;
- un organe de préhension, agencé pour recouvrir la tête de piston.

Ainsi, un avantage procuré par l'organe de préhension est d'améliorer le confort du professionnel de santé lors de l'injection en évitant un contact direct avec la tête de piston rigide.

Selon une caractéristique de l'invention :
- l'organe de préhension comporte une zone rigide et une zone déformable ;
- la partie rigide du poussoir manuel est formée par la tête de piston et par la zone rigide de l'organe de préhension ;
- la partie déformable du poussoir manuel est formée par la zone déformable de l'organe de préhension.

Selon une caractéristique de l'invention :
- l'organe de préhension comporte une zone déformable ;
- la partie rigide du poussoir manuel est formée par la tête de piston ;
- la partie déformable du poussoir manuel est formée par la zone déformable de l'organe de préhension.

Selon une caractéristique de l'invention, la longueur de la tige de piston est comprise entre 40 mm et 120 mm.

### Définitions

- Par « partie rigide », on entend une partie du poussoir qui est rigide (i.e. non-déformable) dans des conditions normales d'utilisation, par exemple à température ambiante (entre 20°C et 30°C) et sous pression atmosphérique.
- Par « partie déformable », on entend une partie du poussoir qui est conçue pour se déformer dans des conditions normales d'utilisation, par exemple à température ambiante (entre 20°C et 30°C) et sous pression atmosphérique.
- Par « se fléchir », on entend la capacité de se déformer par une déformation de type flexion, c'est-à-dire la déformation obtenue par l'action d'une charge se traduisant par une courbure. La déformation de type flexion peut être élastique, c'est-à-dire que la partie déformable peut retrouver sa forme d'origine après avoir été déformé (déformation réversible). La déformation de type flexion peut être plastique (irréversible), auquel cas la seringue manuelle est à usage unique.
- Par « flèche », on entend la valeur maximale du déplacement de la partie déformable suivant un axe parallèle à l'axe longitudinal de la tige de piston.
- Les valeurs X et Y exprimées à l'aide des expressions « entre X et Y » ou « compris entre X et Y » sont incluses dans la plage de valeurs définie.
- Par « module d'Young », on entend le module d'Young *stricto sensu* ou le module à 100% lorsque le premier matériau est un élastomère.
- Par « réseau de motifs périodiques », on entend des motifs espacés selon un intervalle régulier de distance (période spatiale) lorsque la partie déformable est dans la position de repos.
- Par « souple », on entend que le premier matériau est flexible dans des conditions normales d'utilisation, par exemple à température ambiante (entre 20°C et 30°C) et sous pression atmosphérique.
- Par « forme en U inversé », on entend le symétrique d'une forme en « U » selon un axe horizontal.
- Par « forme en V inversé », on entend le symétrique d'une forme en « V » selon un axe horizontal.
- Par « constituée », on entend que la tête de piston est le seul et unique élément composant le poussoir manuel.

### Brève description des dessins

D'autres caractéristiques et avantages apparaîtront dans l'exposé détaillé de différents modes de réalisation de l'invention, l'exposé étant assorti d'exemples et de références aux dessins joints.
Figure 1 est une vue schématique en perspective éclatée d'une seringue manuelle selon l'invention.
Figure 1b is est une vue schématique analogue à la figure 1 où la partie rigide du poussoir manuel est formée par la tête de piston.
Figure 2 est une vue schématique en perspective d'une seringue manuelle selon l'invention, illustrant une partie déformable du poussoir manuel en position de repos.
Figure 3 est une vue schématique analogue à la figure 2, illustrant la partie déformable du poussoir manuel en position fléchie.
Figure 4 est une vue schématique analogue à la figure 3, la partie déformable étant vue sous un autre angle.
Figure 5 est une vue schématique partielle en perspective d'une seringue manuelle selon l'invention, illustrant une tige de piston et un poussoir manuel.

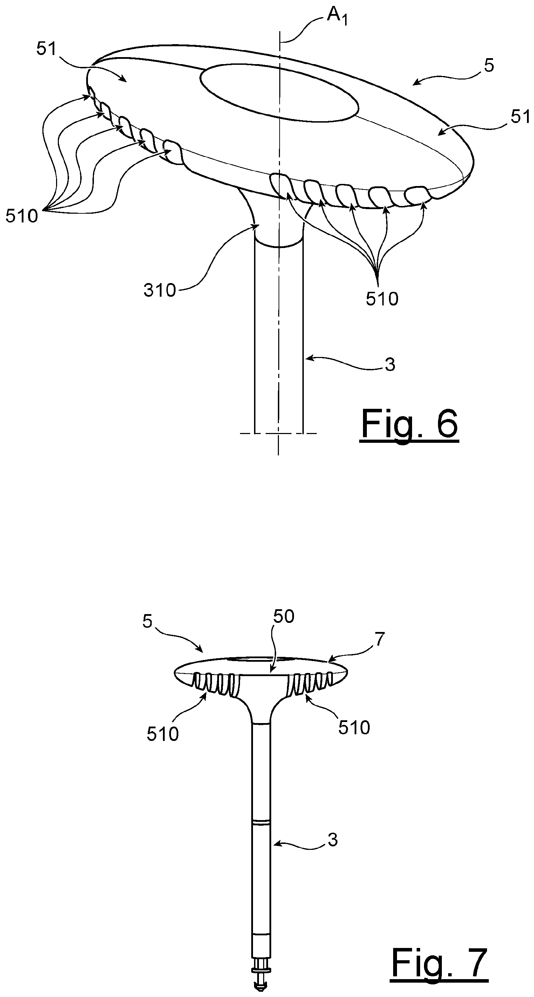

Figure 6 est une vue schématique à l'échelle agrandie de la tige de piston et du poussoir manuel illustrés à la figure 5.
Figure 7 est une vue schématique partielle en perspective d'une seringue manuelle selon l'invention, illustrant une tige de piston et un poussoir manuel réalisés dans des matériaux différents, la partie déformable du poussoir manuel étant en position de repos.

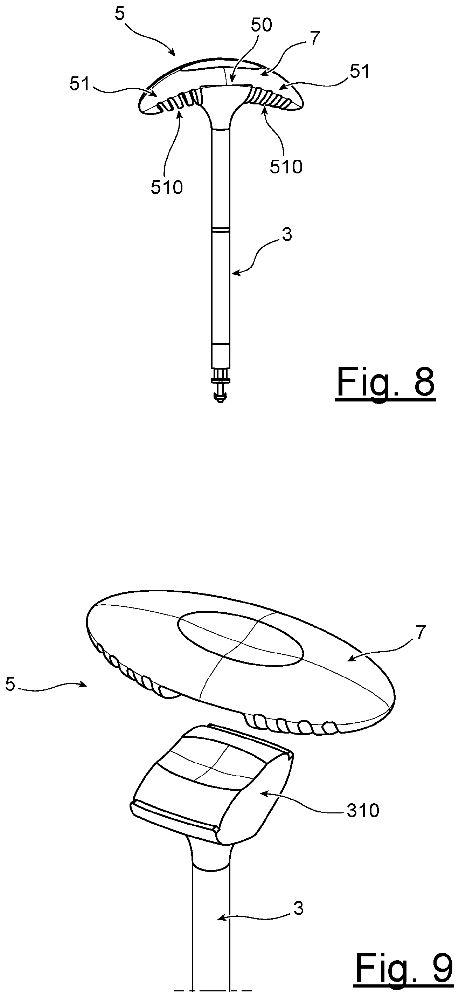

Figure 8 est une vue schématique analogue à la figure 7, la partie déformable du poussoir manuel étant en position fléchie.
Figure 9 est une vue schématique partielle en perspective éclatée d'une seringue manuelle selon l'invention, illustrant une tête de piston et un organe de préhension agencé pour recouvrir la tête de piston.
Figure 10 est une vue schématique en coupe illustrant la partie déformable du poussoir manuel en position de repos et en position fléchie.
Figure 11 est une vue schématique en perspective à l'échelle agrandie d'un poussoir manuel comportant une partie déformable en position de repos.
Figure 12 est une vue schématique en perspective d'un organe de préhension destiné à recouvrir une tête de piston, l'organe de préhension comportant une zone déformable en position de repos.
Figure 13 est une vue schématique en perspective d'ailettes de préhension.
Figure 14 est une vue schématique en perspective illustrant la fixation des ailettes de préhension à la seconde extrémité du corps de seringue.
Figure 15 est une vue schématique en perspective illustrant l'insertion de la tige de piston à travers les ailettes de préhension.
Figure 16 est une vue schématique de dessus d'un poussoir manuel, illustrant une zone centrale rigide et deux zones latérales déformables.
Figure 17 est une vue schématique de dessus d'un poussoir manuel, illustrant une zone centrale rigide et une zone périphérique déformable.
Figure 18 est une vue schématique partielle en perspective d'une seringue manuelle selon l'invention, illustrant une partie inférieure d'un poussoir manuel muni d'organes de butée.

Il est à noter que les dessins décrits ci-avant sont schématiques, et ne sont pas nécessairement à l'échelle par souci de lisibilité et pour en simplifier leur compréhension.

### Exposé détaillé des modes de réalisation

Les éléments identiques ou assurant la même fonction porteront les mêmes références pour les différents modes de réalisation, par souci de simplification.

Un objet de l'invention est une seringue manuelle, comportant :
- un corps 1 de seringue, destiné à recevoir un produit à injecter ; le corps 1 de seringue présentant une première extrémité 10, destinée à être équipée d'un porte-aiguille, et une seconde extrémité 11 opposée ;
- des ailettes 2 de préhension, agencées à la seconde extrémité 11 du corps 1 de seringue ;
- une tige 3 de piston, montée coulissante à l'intérieur du corps 1 de seringue, la tige 3 de piston présentant des première et seconde extrémités 30, 31 opposées ; la tige 3 de piston possédant une longueur L₁ ;
- un butoir 4, agencé à la première extrémité 30 de la tige 3 de piston ;
- un poussoir 5 manuel, agencé à la seconde extrémité 31 de la tige 3 de piston, et sur lequel est exercée manuellement une pression d'injection adaptée pour injecter le produit ;
remarquable en ce que le poussoir 5 manuel comprend :
- une partie rigide 50 ;
- une partie déformable 51, conçue pour se fléchir vers la tige 3 de piston selon une flèche δ comprise entre 5% et 20% de la longueur L₁ de la tige 3 de piston, lorsque la pression d'injection est exercée manuellement sur le poussoir 5 manuel.

La partie déformable 51 peut être conçue pour se fléchir vers la tige 3 de piston selon une flèche δ comprise entre 5% et 25% de la longueur L₁ de la tige 3 de piston, lorsque la pression d'injection est exercée manuellement sur le poussoir 5 manuel. La flèche δ est avantageusement comprise entre 10% et 25%, plus préférentiellement comprise entre 15% et 25% de la longueur L₁ de la tige 3 de piston, lorsque la pression d'injection est exercée manuellement sur le poussoir 5 manuel.

### Corps de seringue

Le corps 1 de seringue est destiné à recevoir un produit à injecter. Le produit à injecter est de préférence un produit visqueux, plus préférentiellement un gel viscoélastique, encore plus préférentiellement un gel viscoélastique à base d'acide hyaluronique. Le corps 1 de seringue est de préférence cylindrique.

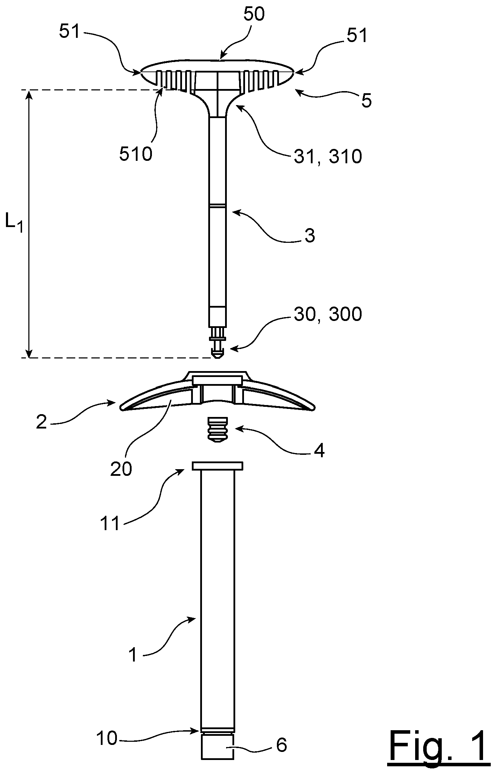

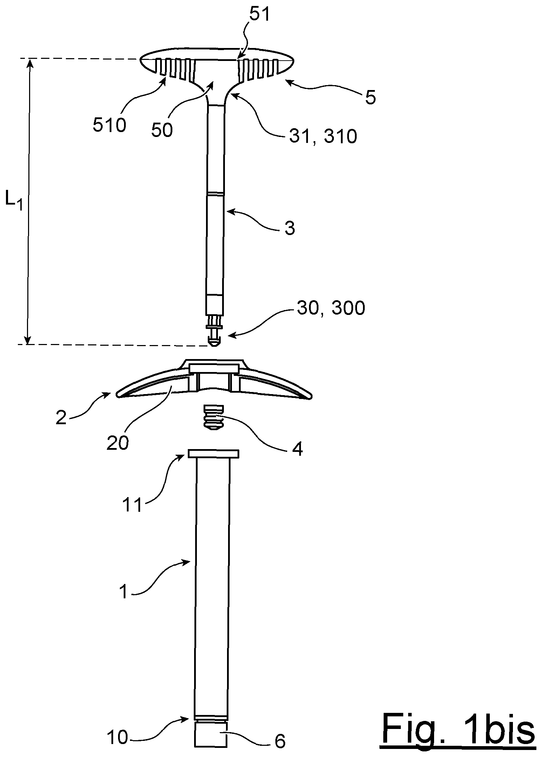

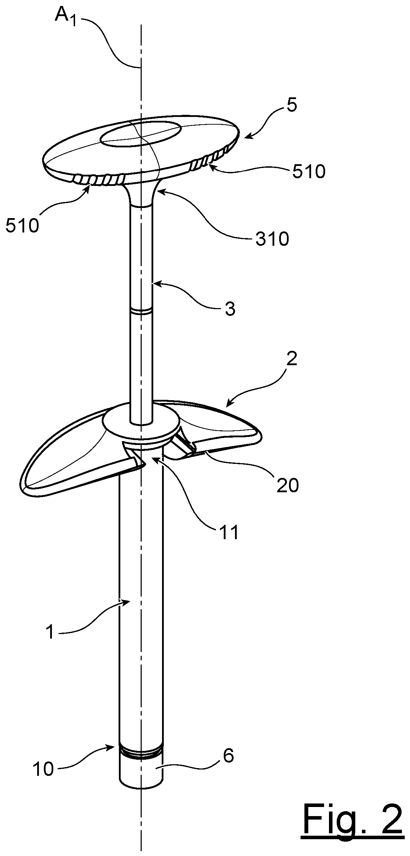

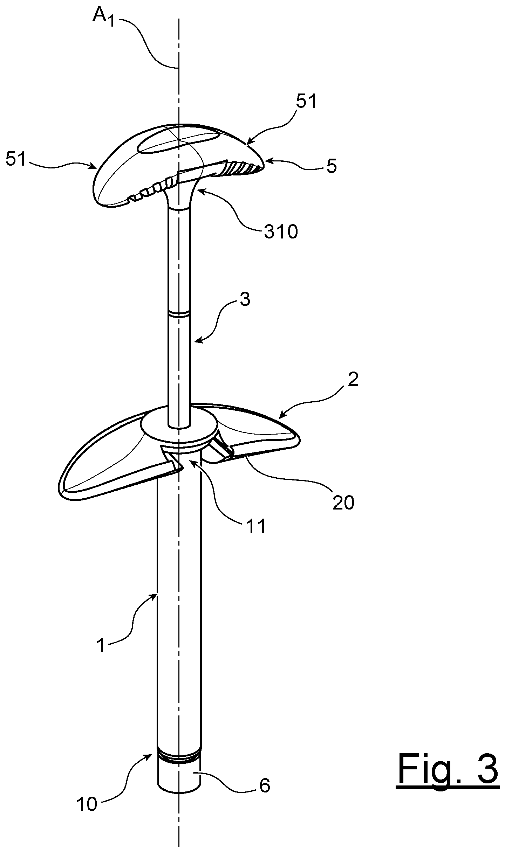

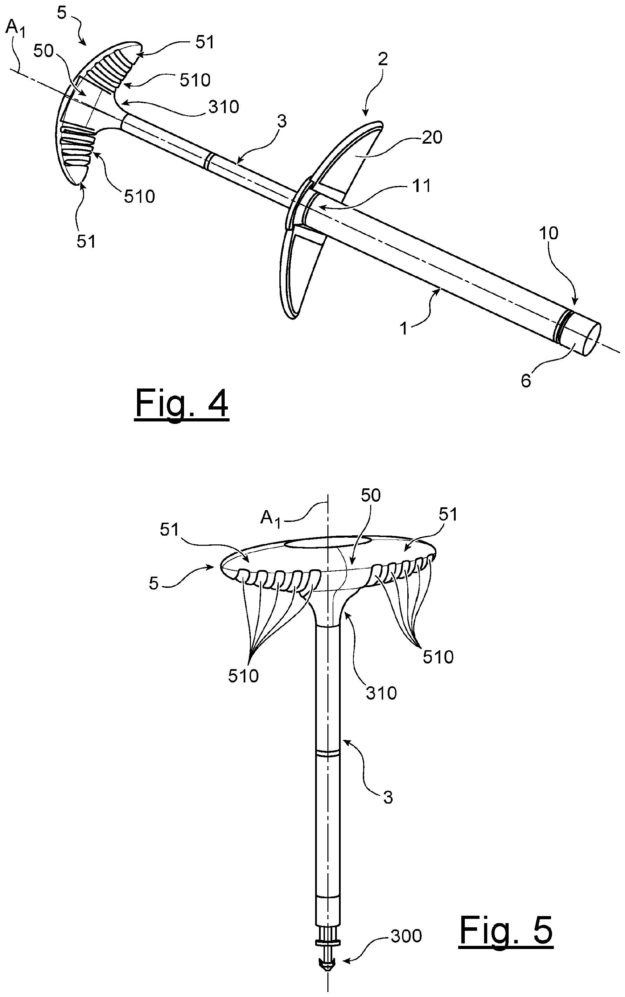

Le corps 1 de seringue présente une première extrémité 10 destinée à être équipée d'un porte-aiguille. Le corps 1 de seringue présente une seconde extrémité 11, opposée à la première extrémité 10. Les première et seconde extrémités 10, 11 du corps 1 de seringue sont ouvertes. La première extrémité 10 du corps 1 de seringue est ouverte pour recevoir le porte-aiguille. Comme illustré aux figures 1 à 4, en l'absence de porte-aiguille, la première extrémité 10 du corps 1 de seringue peut être obstruée par un capuchon 6. La seconde extrémité 11 du corps 1 de seringue est ouverte pour recevoir la tige 3 de piston.

Le corps 1 de seringue est avantageusement réalisé dans un premier matériau plastique, le premier matériau plastique étant de préférence le polycarbonate.

### Ailettes de préhension

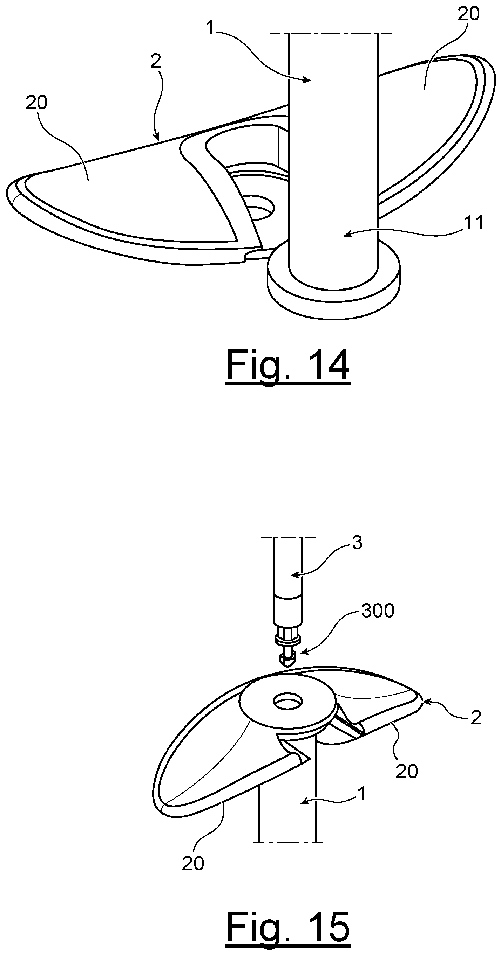

Les ailettes 2 de préhension sont agencées à la seconde extrémité 11 du corps 1 de seringue. Comme illustré à la figure 14, les ailettes 2 de préhension peuvent être montées à la seconde extrémité 11 du corps 1 de seringue par une liaison encastrement.

Les ailettes 2 de préhension présentent une surface de préhension 20 possédant avantageusement une aire comprise entre 400 mm² et 900 mm², de préférence comprise entre 700 mm² et 800 mm². La surface de préhension 20 est avantageusement concave, orientée vers le corps 1 de seringue.

Les ailettes de préhension 2 forment des parties latérales de la seringue s'étendant en saillie du corps 1 de seringue. Les ailettes de préhension 2 sont avantageusement monobloc. Les ailettes de préhension 2 s'étendent suivant un axe longitudinal, et présentent avantageusement un profil transversal (à l'axe longitudinal) formant une surface convexe.

Les ailettes de préhension 2 sont avantageusement réalisées au moins partiellement dans le premier matériau plastique. La surface de préhension 20 des ailettes 2 de préhension est avantageusement réalisée au moins partiellement dans un second matériau plastique, différent du premier matériau plastique. Le second matériau plastique possède avantageusement une dureté comprise entre 30 Shore A et 70 Shore A, de préférence comprise entre 50 Shore A et 70 Shore A. A titre d'exemples non limitatifs, le second matériau plastique peut être un thermoplastique de type SEBS i.e. polystyrène-b-poly(éthylène-butylène)-b-polystyrène, ou le silicone.

### Tige de piston

La tige 3 de piston est montée coulissante à l'intérieur du corps 1 de seringue. La tige 3 de piston présente des première et seconde extrémités 30, 31 opposées. La tige 3 de piston comporte avantageusement une tête d'encliquetage 300 agencée à la première extrémité 30 de la tige 3 de piston. La tige 3 de piston comporte avantageusement une tête de piston 310 agencée à la seconde extrémité 31 de la tige 3 de piston. La tête de piston 310 et la tige 3 de piston sont avantageusement monobloc. La tige 3 de piston est préférentiellement réalisée dans un matériau plastique, plus préférentiellement dans un matériau thermoplastique. A titre d'exemple non limitatif, la tige 3 de piston peut être réalisée en polycarbonate.

La tige 3 de piston s'étend suivant un premier axe longitudinal A₁. La longueur L₁ de la tige 3 de piston (suivant le premier axe longitudinal A₁) est préférentiellement comprise entre 40 mm et 120 mm. Comme illustré à la figure 1, la longueur L₁ de la tige 3 de piston est définie entre l'extrémité de la tête de piston 310 et l'extrémité de la tête d'encliquetage 300. Le ratio entre la largeur de la tige 3 de piston et la longueur L₁ de la tige 3 de piston peut être compris entre 2% et 30%, préférentiellement compris entre 3% et 20%, plus préférentiellement compris entre 4% et 10%, encore plus préférentiellement compris entre 5,5% et 8,5%.

### Butoir

Le butoir 4 (illustré à la figure 1) est agencé à la première extrémité 30 de la tige 3 de piston. Le butoir 4 peut présenter une paroi interne, élastiquement déformable, et délimitant une cavité. Par « paroi interne », on entend la surface interne du butoir 4 délimitant une cavité, le butoir 4 étant creux. Par « élastiquement déformable », on entend que la paroi interne du butoir 4 peut se déformer de manière réversible sous l'action de contraintes mécaniques (inférieures à la limite élastique), c'est-à-dire que la paroi interne du butoir 4 peut revenir à sa forme initiale lorsque les contraintes mécaniques cessent. La tête d'encliquetage 300 est avantageusement encliquetée à l'intérieur de la cavité du butoir 4. Par « encliquetée », on entend un emboîtage élastique entre la tête d'encliquetage 300 et la paroi interne du butoir 4, à l'intérieur de la cavité du butoir 4. Pour ce faire, la tête d'encliquetage 300 peut comporter:
- une surface d'appui plane, venant en appui contre la paroi interne du butoir 4 lorsque la tige 3 de piston coulisse suivant le sens opposé au sens d'injection du produit ;
- au moins un élément saillant, s'étendant en saillie de la surface d'appui plane, et agencé pour pénétrer dans la paroi interne du butoir 4 lorsque la tige 3 de piston coulisse suivant le sens opposé au sens d'injection du produit.

Le butoir 4 est préférentiellement réalisé dans un matériau élastomère, de préférence un caoutchouc butyle chloré ou bromé.

### Poussoir manuel

Le poussoir 5 manuel est agencé à la seconde extrémité 31 de la tige 3 de piston. Une pression d'injection est exercée manuellement sur le poussoir 5 manuel par le professionnel de santé, la pression d'injection étant adaptée pour injecter le produit.

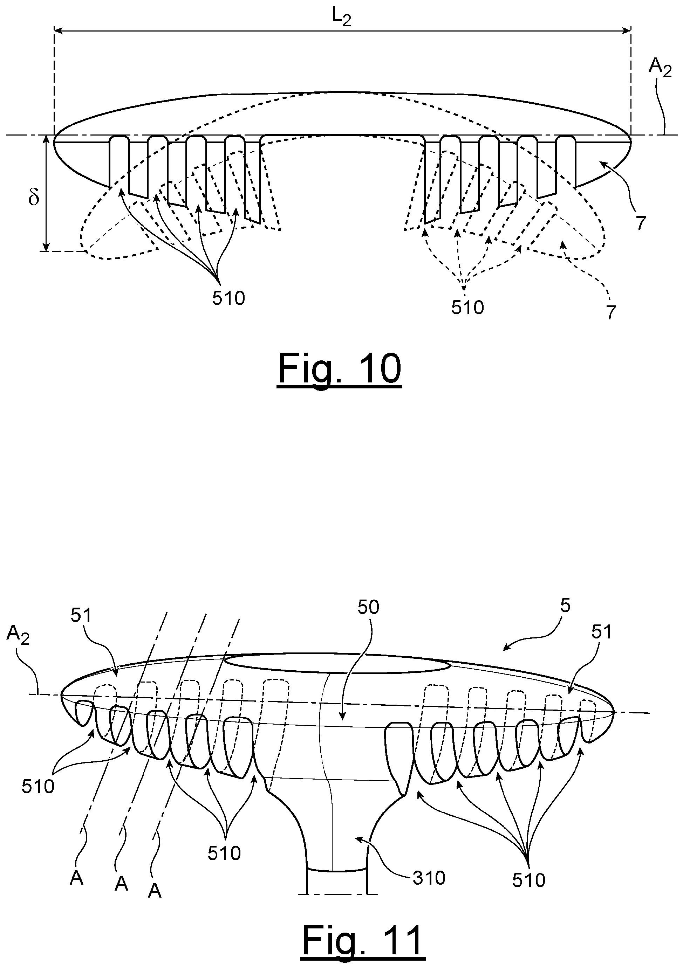

Le poussoir 5 manuel comprend :
- une partie rigide 50 ;
- une partie déformable 51, conçue pour se fléchir vers la tige 3 de piston selon une flèche δ (illustrée à la figure 10) comprise entre 5% et 20% de la longueur L₁ de la tige 3 de piston, lorsque la pression d'injection est exercée manuellement sur le poussoir 5 manuel.

La partie déformable 51 peut être conçue pour se fléchir vers la tige 3 de piston selon une flèche δ comprise entre 5% et 25% de la longueur L₁ de la tige 3 de piston, lorsque la pression d'injection est exercée manuellement sur le poussoir 5 manuel. La flèche δ est avantageusement comprise entre 10% et 25%, plus préférentiellement comprise entre 15% et 25% de la longueur L₁ de la tige 3 de piston, lorsque la pression d'injection est exercée manuellement sur le poussoir 5 manuel.

La flèche δ est la valeur maximale du déplacement de la partie déformable 51 suivant un axe parallèle au premier axe longitudinal A₁ de la tige 3 de piston.

Le poussoir 5 manuel s'étend préférentiellement suivant un deuxième axe longitudinal A₂, perpendiculaire au premier axe longitudinal A₁. Le poussoir 5 manuel possède une longueur L₂ suivant le deuxième axe longitudinal A₂.

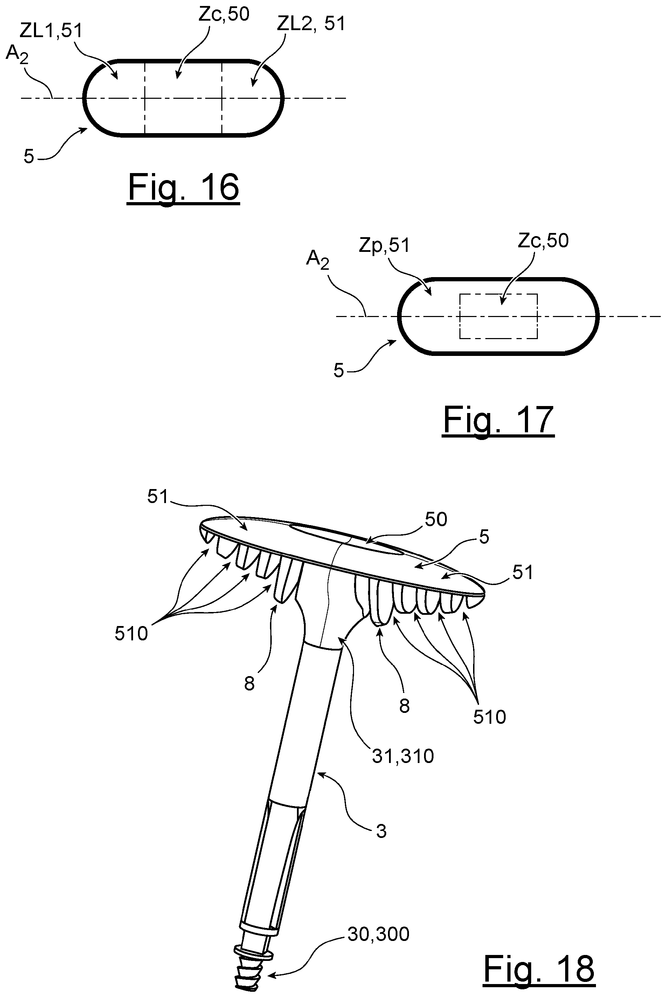

Selon une première possibilité illustrée à la figure 16 :
- le poussoir 5 manuel comporte successivement une première zone latérale ZL1, une zone centrale Zc et une seconde zone latérale ZL2 suivant le deuxième axe longitudinal A₂ ;
- la zone centrale Zc forme la partie rigide 50 ;
- la première zone latérale ZL1 et/ou la seconde zone latérale ZL2 forment la partie déformable 51.

Selon une deuxième possibilité illustrée à la figure 17 :
- le poussoir 5 manuel comporte une zone centrale Zc et une zone périphérique Zp s'étendant autour de la zone centrale Zc ;
- la zone centrale Zc forme la partie rigide 50 ;
- la zone périphérique Zp forme la partie déformable 51.

Le poussoir 5 manuel peut comporter :
- une partie supérieure sur laquelle est exercée manuellement une pression d'injection adaptée pour injecter le produit ;
- une partie inférieure, opposée à la partie supérieure.

Le terme « supérieure » est choisi par convention pour désigner la partie sur laquelle est exercée manuellement une pression d'injection pour injecter le produit, quelle que soit la position de préhension. En effet, dans la majorité des positions de préhension, l'injection du produit est exécutée de haut en bas. Il est à noter que dans certaines positions de préhension, l'injection du produit peut être exécutée du bas vers le haut ou encore latéralement.

La partie supérieure du poussoir 5 manuel possède avantageusement une surface incurvée adaptée pour épouser une forme palmaire d'une main. La surface incurvée, adaptée pour épouser une forme palmaire d'une main, comporte avantageusement :
- une partie centrale rigide ;
- au moins une partie latérale déformable ou une partie périphérique déformable.

La partie déformable 51 du poussoir 5 manuel est mobile entre une position de repos et une position fléchie dans laquelle la partie déformable 51 est fléchie vers la tige 3 de piston selon la flèche δ comprise entre 5% et 20% de la longueur L₁ de la tige 3 de piston. La partie déformable 51 du poussoir 5 manuel peut être mobile entre une position de repos et une position fléchie dans laquelle la partie déformable 51 est fléchie vers la tige 3 de piston selon une flèche δ comprise entre 5% et 25% de la longueur L₁ de la tige 3 de piston. La flèche δ est avantageusement comprise entre 10% et 25%, plus préférentiellement comprise entre 15% et 25% de la longueur L₁ de la tige 3 de piston, lorsque la pression d'injection est exercée manuellement sur le poussoir 5 manuel. La partie déformable 51 du poussoir 5 manuel et la partie rigide 50 du poussoir 5 manuel demeurent en contact lorsque la partie déformable 51 du poussoir 5 manuel est déplacée de la position de repos vers la position fléchie, et de la position fléchie vers la position de repos. En particulier, la partie déformable 51 du poussoir 5 manuel et la partie rigide 50 du poussoir 5 manuel sont en contact lorsque la partie déformable 51 du poussoir 5 manuel est dans la position de repos. La partie déformable 51 du poussoir 5 manuel et la partie rigide 50 du poussoir 5 manuel peuvent être jointives dans un plan de jonction lorsque la partie déformable 51 est dans la position de repos, le plan de jonction étant perpendiculaire au premier axe longitudinal A₁. En d'autres termes, la partie déformable 51 du poussoir 5 manuel et la partie rigide 50 du poussoir 5 manuel sont dépourvues d'une distance d'espacement s'étendant suivant le premier axe longitudinal A₁. Plus précisément, la partie déformable 51 du poussoir 5 manuel et la partie rigide 50 du poussoir 5 manuel sont dépourvues d'une distance d'espacement s'étendant suivant le premier axe longitudinal A₁ quelle que soit la position de la partie déformable 51 du poussoir 5 manuel.

### Mode de réalisation n°1 : présence de zones d'affaiblissement

La partie déformable 51 est réalisée dans un premier matériau possédant un premier module d'Young. La partie déformable 51 comporte des zones d'affaiblissement 510 du premier matériau, agencées en fonction du premier module d'Young pour fléchir le premier matériau vers la tige 3 de piston selon la flèche δ comprise entre 5% et 20% de la longueur L₁ de la tige 3 de piston, lorsque la pression d'injection est exercée manuellement sur le poussoir 5 manuel. Les zones d'affaiblissement 510 du premier matériau peuvent être agencées en fonction du premier module d'Young pour fléchir le premier matériau vers la tige 3 de piston selon une flèche δ comprise entre 5% et 25% (préférentiellement entre 10% et 25%, plus préférentiellement entre 15% et 25%) de la longueur L₁ de la tige 3 de piston, lorsque la pression d'injection est exercée manuellement sur le poussoir 5 manuel.

La partie déformable 51 du poussoir 5 manuel est mobile entre une position de repos et une position fléchie dans laquelle la partie déformable 51 est fléchie vers la tige 3 de piston selon la flèche δ comprise entre 5% et 20% de la longueur L₁ de la tige 3 de piston. La partie déformable 51 du poussoir 5 manuel peut être mobile entre une position de repos et une position fléchie dans laquelle la partie déformable 51 est fléchie vers la tige 3 de piston selon une flèche δ comprise entre 5% et 25% de la longueur L₁ de la tige 3 de piston. La flèche δ est avantageusement comprise entre 10% et 25%, plus préférentiellement comprise entre 15% et 25% de la longueur L₁ de la tige 3 de piston, lorsque la pression d'injection est exercée manuellement sur le poussoir 5 manuel.

Chaque zone d'affaiblissement 510 du premier matériau forme avantageusement un évidement de matière, l'évidement de matière formant de préférence une rainure au sein du premier matériau.

Les zones d'affaiblissement 510 du premier matériau sont avantageusement agencées pour former un réseau de motifs périodiques. Plus précisément, les zones d'affaiblissement 510 du premier matériau sont avantageusement agencées pour former un réseau de motifs périodiques lorsque la partie déformable 51 du poussoir 5 manuel est dans la position de repos. Le nombre de motifs périodiques, la période spatiale des motifs périodiques et la répartition spatiale des motifs périodiques sont ajustés en fonction du premier module d'Young afin d'obtenir la flèche δ vers la tige 3 de piston, la flèche δ étant comprise entre 5% et 20% de la longueur L₁ de la tige 3 de piston, lorsque la pression d'injection est exercée manuellement sur le poussoir 5 manuel. Le nombre de motifs périodiques, la période spatiale des motifs périodiques et la répartition spatiale des motifs périodiques sont ajustés en fonction du premier module d'Young afin d'obtenir la flèche δ vers la tige 3 de piston, la flèche δ pouvant être comprise entre 5% et 25% de la longueur L₁ de la tige 3 de piston, lorsque la pression d'injection est exercée manuellement sur le poussoir 5 manuel. La flèche δ est avantageusement comprise entre 10% et 25%, plus préférentiellement comprise entre 15% et 25% de la longueur L₁ de la tige 3 de piston, lorsque la pression d'injection est exercée manuellement sur le poussoir 5 manuel.

Comme illustré à la figure 11, les zones d'affaiblissement 510 s'étendent avantageusement suivant des axes A parallèles entre eux lorsque la partie déformable 51 est dans la position de repos.

Les zones d'affaiblissement 510 du premier matériau s'étendent avantageusement chacune suivant un axe A perpendiculaire aux premier et deuxième axes longitudinaux A₁, A₂ lorsque la partie déformable 51 est dans la position de repos.

Les zones d'affaiblissement 510 du premier matériau présentent une section longitudinale, suivant le premier axe longitudinal A₁, définissant avantageusement une forme en U inversé ou en V inversé lorsque la partie déformable 51 est dans la position de repos et lorsque le premier axe longitudinal A₁ est un axe vertical, la seconde extrémité 31 de la tige 3 de piston étant située au-dessus de la première extrémité 30 de la tige 3 de piston par rapport à l'axe vertical.

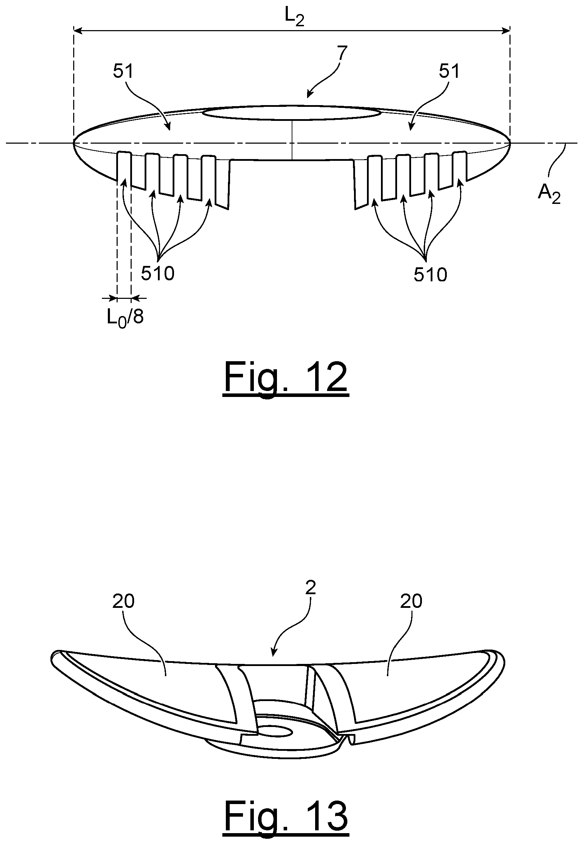

Comme illustré à la figure 12, les zones d'affaiblissement 510 du premier matériau possèdent une longueur totale L₀, suivant le deuxième axe longitudinal A₂, avantageusement comprise entre 30% et 50% de la longueur L₂ du poussoir 5 manuel, lorsque la partie déformable 51 est dans la position de repos. La longueur totale Lₐ des zones d'affaiblissement 510, suivant le deuxième axe longitudinal A₂, correspond à la longueur cumulée des zones d'affaiblissement 510 (e.g. évidements de matière, motifs périodiques creux). A titre d'exemple non limitatif, huit zones d'affaiblissement 510, de longueur identique suivant le deuxième axe longitudinal A₂, sont représentées à la figure 12, chaque zone d'affaiblissement possédant une longueur L_{0/}8 suivant le deuxième axe longitudinal A₂. A titre d'exemple non limitatif, chaque zone d'affaiblissement 510 peut posséder une longueur suivant le deuxième axe longitudinal A₂ comprise entre 1 mm et 2 mm.

Le premier matériau peut être rigide, et la partie rigide du poussoir 5 manuel est réalisée dans le premier matériau. Le premier matériau rigide est de préférence un polymère, plus préférentiellement un polymère choisi parmi le polypropylène, l'acrylonitrile butadiène styrène, un polyamide, le polyéthylène. Le premier matériau rigide possède préférentiellement un module d'Young compris entre 900 MPa et 1600 MPa.

Selon une variante de réalisation, le premier matériau est souple. Le premier matériau souple est de préférence un élastomère, plus préférentiellement un élastomère thermoplastique. Le premier matériau souple est par exemple choisi parmi un caoutchouc, le polychloroprène (Néoprène^{™}), le silicone, le nylon. Le premier matériau souple possède préférentiellement un module à 100% compris entre 1 MPa et 2 MPa.

### Mode de réalisation n°2 : absence de zones d'affaiblissement

La partie déformable 51 du poussoir 5 manuel est réalisée dans un matériau souple possédant un module d'Young adapté pour fléchir le matériau souple vers la tige 3 de piston selon la flèche δ comprise entre 5% et 20% de la longueur L₁ de la tige 3 de piston, lorsque la pression d'injection est exercée manuellement sur le poussoir 5 manuel. La partie déformable 51 du poussoir 5 manuel peut être réalisée dans un matériau souple possédant un module d'Young adapté pour fléchir le matériau souple vers la tige 3 de piston selon une flèche δ comprise entre 5% et 25% (préférentiellement entre 10% et 25%, plus préférentiellement entre 15% et 25%) de la longueur L₁ de la tige 3 de piston, lorsque la pression d'injection est exercée manuellement sur le poussoir 5 manuel. La partie déformable 51 du poussoir 5 manuel est alors dépourvue de zones 510 d'affaiblissement du matériau souple. L'homme du métier peut ajuster la longueur de la partie déformable 51 du poussoir 5 manuel suivant le deuxième axe longitudinal A₂ en fonction du module d'Young du matériau souple afin d'obtenir la flèche δ vers la tige 3 de piston, la flèche δ étant comprise entre 5% et 20% de la longueur L₁ de la tige 3 de piston, lorsque la pression d'injection est exercée manuellement sur le poussoir 5 manuel. L'homme du métier peut ajuster la longueur de la partie déformable 51 du poussoir 5 manuel suivant le deuxième axe longitudinal A₂ en fonction du module d'Young du matériau souple afin d'obtenir la flèche δ vers la tige 3 de piston, la flèche δ étant comprise entre 5% et 25% (préférentiellement entre 10% et 25%, plus préférentiellement entre 15% et 25%) de la longueur L₁ de la tige 3 de piston, lorsque la pression d'injection est exercée manuellement sur le poussoir 5 manuel.

A titre d'exemples non limitatifs, le matériau souple peut être choisi parmi un polyuréthane, le polystyrène expansé, le liège.

### Mode réalisation n°3 : poussoir constitué de la tête de piston

Le poussoir 5 manuel peut être constitué d'une tête de piston 310 formée à la seconde extrémité 31 de la tige 3 de piston. La tête de piston 310 comprend alors la partie rigide 50 et la partie déformable 51 du poussoir 5 manuel. La seringue manuelle est alors dépourvue d'un organe de préhension 7 agencé pour recouvrir la tête de piston 310. La tête de piston 310, constituant le poussoir 5 manuel, assure la préhension de la seringue avec les ailettes 2 de préhension.

### Mode de réalisation n°4 : poussoir comportant la tête de piston et un organe de préhension

Le poussoir 5 manuel peut comporter :
- une tête de piston 310, rigide, formée à la seconde extrémité 31 de la tige 3 de piston ;
- un organe de préhension 7, agencé pour recouvrir la tête de piston 310.

L'organe de préhension 7 et la tête de piston 310 rigide peuvent être monobloc. Selon une alternative, l'organe de préhension 7 peut être monté sur la tête de piston 310 par une liaison encastrement ou par un emboîtage élastique.

Selon une première possibilité :
- l'organe de préhension 7 comporte une zone rigide et une zone déformable ;
- la partie rigide 50 du poussoir 5 manuel est formée par la tête de piston 310 et par la zone rigide de l'organe de préhension 7 ;
- la partie déformable 51 du poussoir 5 manuel est formée par la zone déformable de l'organe de préhension 7.

La zone rigide de l'organe de préhension 7 et la tête de piston 310 rigide peuvent être réalisées dans des matériaux identiques ou différents. Lorsque la zone rigide de l'organe de préhension 7 et la tête de piston 310 sont réalisées dans des matériaux identiques et lorsque l'organe de préhension 7 et la tête de piston 310 sont monobloc, l'organe de préhension 7 et la tête de piston 310 peuvent être obtenus par un moulage bi-injection (bi-matière), la première matière étant le matériau de la tête de piston 310 et de la zone rigide de l'organe de préhension 7, la deuxième matière étant le matériau de la zone déformable de l'organe de préhension 7. Lorsque l'organe de préhension 7 et la tête de piston 310 ne sont pas monobloc, l'organe de préhension 7 peut être obtenu par un moulage bi-injection (bi-matière), la première matière étant le matériau de la zone rigide de l'organe de préhension 7, la deuxième matière étant le matériau de la zone déformable de l'organe de préhension 7.

Selon une deuxième possibilité :
- l'organe de préhension 7 comporte une zone déformable ;
- la partie rigide 50 du poussoir 5 manuel est formée par la tête de piston 310 ;
- la partie déformable 51 du poussoir 5 manuel est formée par la zone déformable de l'organe de préhension 7.

Lorsque l'organe de préhension 7 et la tête de piston 310 sont monobloc, l'organe de préhension 7 et la tête de piston 310 peuvent être obtenus par un moulage bi-injection (bi-matière), la première matière étant le matériau de la tête de piston 310, la deuxième matière étant le matériau de l'organe de préhension 7.

### Organes de butée

La partie inférieure du poussoir 5 manuel comporte avantageusement deux organes de butée 8 agencés de part et d'autre du premier axe longitudinal A₁ de manière à bloquer un glissement de doigts suivant le deuxième axe longitudinal A₂. Les organes de butée 8 possèdent avantageusement une hauteur (dimension suivant le premier axe longitudinal A₁) comprise entre 8 mm et 12 mm. Les organes de butée 8 sont avantageusement espacés de la tige 3 de piston d'une distance (dimension suivant le deuxième axe longitudinal A₂) comprise entre 1,5 mm et 2 mm lorsque la partie déformable 51 du poussoir 5 manuel est dans la position de repos.

Comme illustré à la figure 18, lorsque les zones d'affaiblissement 510 forment des évidements de matière délimitant une alternance de motifs creux et de motifs pleins, les organes de butée 8 forment avantageusement des motifs pleins s'étendant en saillie des motifs pleins adjacents de manière à bloquer un glissement de doigts suivant le deuxième axe longitudinal A₂. Plus précisément, les organes de butée 8 s'étendent en saillie des motifs pleins adjacents suivant le premier axe longitudinal A₁. Les motifs pleins possèdent avantageusement une hauteur (dimension suivant le premier axe longitudinal A₁) décroissante du centre de poussoir 5 manuel vers un bord du poussoir 5 manuel (suivant le deuxième axe longitudinal A₂).

L'invention ne se limite pas aux modes de réalisation exposés. L'homme du métier est mis à même de considérer leurs combinaisons techniquement opérantes, et de leur substituer des équivalents.

## Revendications

1. Seringue manuelle, comportant :
- un corps (1) de seringue, destiné à recevoir un produit à injecter ; le corps (1) de seringue présentant une première extrémité (10), destinée à être équipée d'un porte-aiguille, et une seconde extrémité (11) opposée ;
- des ailettes (2) de préhension, agencées à la seconde extrémité (11) du corps (1) de seringue ;
- une tige (3) de piston, montée coulissante à l'intérieur du corps (1) de seringue, la tige (3) de piston présentant des première et seconde extrémités (30, 31) opposées ; la tige (3) de piston possédant une longueur (L₁) ; la tige (3) de piston s'étend suivant un premier axe longitudinal (A₁) ;
- un butoir (4), agencé à la première extrémité (30) de la tige (3) de piston ;
- un poussoir (5) manuel, agencé à la seconde extrémité (31) de la tige (3) de piston, et sur lequel est exercée manuellement une pression d'injection adaptée pour injecter le produit ;_le poussoir (5) manuel s'étend suivant un deuxième axe longitudinal (A₂), perpendiculaire au premier axe longitudinal (A₁) ; le poussoir (5) manuel comporte une partie rigide (50), formée par une zone centrale (Zc) ;
**caractérisée en ce que** le poussoir (5) manuel comporte successivement une première zone latérale (ZL1), la zone centrale (Zc) et une seconde zone latérale (ZL2) suivant le deuxième axe longitudinal (A₂) ;
et **en ce que** le poussoir (5) manuel comprend une partie déformable (51), formée par la première zone latérale (ZL1) et/ou la seconde zone latérale (ZL2), conçue pour se fléchir vers la tige (3) de piston selon une flèche (δ) comprise entre 5% et 25% de la longueur (L₁) de la tige (3) de piston, lorsque la pression d'injection est exercée manuellement sur le poussoir (5) manuel.

2. Seringue selon la revendication 1, dans laquelle la partie déformable (51) est réalisée dans un premier matériau possédant un premier module d'Young ; et la partie déformable (51) comporte des zones d'affaiblissement (510) du premier matériau, agencées en fonction du premier module d'Young pour fléchir le premier matériau vers la tige (3) de piston selon la flèche (δ) comprise entre 5% et 25% de la longueur (L₁) de la tige (3) de piston, lorsque la pression d'injection est exercée manuellement sur le poussoir (5) manuel.

3. Seringue selon la revendication 2, dans laquelle chaque zone d'affaiblissement (510) du premier matériau forme un évidement de matière, l'évidement de matière formant de préférence une rainure au sein du premier matériau.

4. Seringue selon la revendication 2 ou 3, dans laquelle les zones d'affaiblissement (510) du premier matériau sont agencées pour former un réseau de motifs périodiques.

5. Seringue selon l'une des revendications 2 à 4, dans laquelle :
- la partie déformable (51) du poussoir (5) manuel est mobile entre une position de repos et une position fléchie dans laquelle la partie déformable (51) est fléchie vers la tige (3) de piston selon la flèche (δ) comprise entre 5% et 25% de la longueur (L₁) de la tige (3) de piston ;
- les zones d'affaiblissement (510) s'étendent suivant des axes (A) parallèles entre eux lorsque la partie déformable (51) est dans la position de repos.

6. Seringue selon l'une des revendications 2 à 5, dans laquelle le premier matériau est rigide, et la partie rigide (50) du poussoir (5) manuel est réalisée dans le premier matériau.

7. Seringue selon l'une des revendications 2 à 5, dans laquelle le premier matériau est souple.

8. Seringue selon la revendication 1, dans laquelle la partie déformable (51) du poussoir (5) manuel est réalisée dans un matériau souple possédant un module d'Young adapté pour fléchir le matériau souple vers la tige (3) de piston selon la flèche (δ) comprise entre 5% et 25% de la longueur (L₁) de la tige (3) de piston, lorsque la pression d'injection est exercée manuellement sur le poussoir (5) manuel.

9. Seringue selon l'une des revendications 2 à 7, dans laquelle :
- la partie déformable (51) du poussoir (5) manuel est mobile entre une position de repos et une position fléchie dans laquelle la partie déformable (51) est fléchie vers la tige (3) de piston selon la flèche (δ) comprise entre 5% et 25% de la longueur (L₁) de la tige (3) de piston ;
- les zones d'affaiblissement (510) du premier matériau s'étendent chacune suivant un axe (A) perpendiculaire aux premier et deuxième axes longitudinaux (A₁, A₂) lorsque la partie déformable (51) est dans la position de repos.

10. Seringue selon l'une des revendications 2 à 7, dans laquelle :
- la partie déformable (51) du poussoir (5) manuel est mobile entre une position de repos et une position fléchie dans laquelle la partie déformable (51) est fléchie vers la tige (3) de piston selon la flèche (δ) comprise entre 5% et 25% de la longueur (L₁) de la tige (3) de piston ;
- les zones d'affaiblissement (510) du premier matériau présentent une section longitudinale, suivant le premier axe longitudinal (A₁), définissant une forme en U inversé ou en V inversé lorsque la partie déformable (51) est dans la position de repos et lorsque le premier axe longitudinal (A₁) est un axe vertical, la seconde extrémité (31) de la tige (3) de piston étant située au-dessus de la première extrémité (30) de la tige (3) de piston par rapport à l'axe vertical.

11. Seringue selon la revendication 9 ou 10, dans laquelle :
- le poussoir (5) manuel possède une longueur (L₂) suivant le deuxième axe longitudinal (A₂) ;
- les zones d'affaiblissement (510) du premier matériau possèdent une longueur totale (L₀), suivant le deuxième axe longitudinal (A₂), comprise entre 30% et 50% de la longueur (L₂) du poussoir (5) manuel, lorsque la partie déformable (51) est dans la position de repos.

12. Seringue selon l'une des revendications 1 à 11, dans laquelle :
- le poussoir (5) manuel comporte une partie supérieure sur laquelle est exercée manuellement une pression d'injection adaptée pour injecter le produit, et une partie inférieure opposée à la partie supérieure ;
- la partie inférieure du poussoir (5) manuel comporte deux organes de butée (8) agencés de part et d'autre du premier axe longitudinal (A₁) de manière à bloquer un glissement de doigts suivant le deuxième axe longitudinal (A₂).

13. Seringue selon l'une des revendications 1 à 12, dans laquelle le poussoir (5) manuel est constitué d'une tête de piston (310) formée à la seconde extrémité (31) de la tige (3) de piston, la tête de piston (310) comprenant la partie rigide (50) et la partie déformable (51).

14. Seringue selon l'une des revendications 1 à 12, dans laquelle le poussoir (5) manuel comporte :
- une tête de piston (310), rigide, formée à la seconde extrémité (31) de la tige (3) de piston ;
- un organe de préhension (7), agencé pour recouvrir la tête de piston (310).

15. Seringue selon la revendication 14, dans laquelle :
- l'organe de préhension (7) comporte une zone rigide et une zone déformable ;
- la partie rigide (50) du poussoir (5) manuel est formée par la tête de piston (310) et par la zone rigide de l'organe de préhension (7) ;
- la partie déformable (51) du poussoir (5) manuel est formée par la zone déformable de l'organe de préhension (7).

16. Seringue selon la revendication 14, dans laquelle :
- l'organe de préhension (7) comporte une zone déformable ;
- la partie rigide (50) du poussoir (5) manuel est formée par la tête de piston (310) ;
- la partie déformable (51) du poussoir (5) manuel est formée par la zone déformable de l'organe de préhension (7).

17. Seringue selon l'une des revendications 1 à 16, dans laquelle la longueur (L₁) de la tige (3) de piston est comprise entre 40 mm et 120 mm.

## Patentansprüche

1. Manuelle Spritze, umfassend:
- einen Spritzenkörper (1), der zur Aufnahme eines zu injizierenden Produkts bestimmt ist; wobei der Spritzenkörper (1) ein erstes Ende (10), das dazu bestimmt ist, mit einem Nadelhalter ausgestattet zu werden, und ein gegenüberliegendes zweites Ende (11) aufweist;
- Greifflügel (2), die am zweiten Ende (11) des Spritzenkörpers (1) angeordnet sind;
- eine Kolbenstange (3), die im Inneren des Spritzenkörpers (1) verschiebbar gelagert ist, wobei die Kolbenstange (3) entgegengesetzte erste und zweite Enden (30, 31) aufweist; wobei die Kolbenstange (3) eine Länge (L₁) aufweist; wobei sich die Kolbenstange (3) entlang einer ersten Längsachse (A₁) erstreckt;
- einen Stopfen (4), der am ersten Ende (30) der Kolbenstange (3) angeordnet ist;
- einen manuellen Drücker (5), der am zweiten Ende (31) der Kolbenstange (3) angeordnet ist und auf den ein zum Injizieren des Produkts geeigneter Injektionsdruck manuell ausgeübt wird; wobei sich der manuelle Drücker (5) entlang einer zweiten Längsachse (A₂), senkrecht zu der ersten Längsachse (A₁), erstreckt; wobei der manuelle Drücker (5) einen starren Abschnitt (50) umfasst, der durch einen zentralen Bereich (Zc) gebildet ist;
**dadurch gekennzeichnet, dass** der manuelle Drücker (5) entlang der zweiten Längsachse (A₂) nacheinander einen ersten Seitenbereich (ZL₁), den zentralen Bereich (Zc) und einen zweiten Seitenbereich (ZL₂) umfasst;
und dass der manuelle Drücker (5) einen verformbaren Abschnitt (51) umfasst, der durch den ersten Seitenbereich (ZL₁) und/oder den zweiten Seitenbereich (ZL₂) gebildet ist und dazu ausgelegt ist, sich zur Kolbenstange (3) hin mit einer Durchbiegung (δ) zwischen 5% und 25% der Länge (L₁) der Kolbenstange (3) zu biegen, wenn der Injektionsdruck manuell auf den manuellen Drücker (5) ausgeübt wird.

2. Spritze nach Anspruch 1, wobei der verformbare Abschnitt (51) aus einem ersten Material mit einem ersten Young'schen Modul gefertigt ist; und wobei der verformbare Abschnitt (51) Schwächungsbereiche (510) des ersten Materials umfasst, die in Abhängigkeit vom ersten Young'schen Modul angeordnet sind, um das erste Material zur Kolbenstange (3) hin mit der Durchbiegung (δ) zwischen 5% und 25% der Länge (L₁) der Kolbenstange (3) zu biegen, wenn der Injektionsdruck manuell auf den manuellen Drücker (5) ausgeübt wird.

3. Spritze nach Anspruch 2, wobei jeder Schwächungsbereich (510) des ersten Materials eine Materialaussparung bildet, wobei die Materialaussparung vorzugsweise eine Nut innerhalb des ersten Materials bildet.

4. Spritze nach Anspruch 2 oder 3, wobei die Schwächungsbereiche (510) des ersten Materials so angeordnet sind, dass sie eine Anordnung periodischer Muster bilden.

5. Spritze nach einem der Ansprüche 2 bis 4, wobei:
- der verformbare Abschnitt (51) des manuellen Drückers (5) zwischen einer Ruhestellung und einer durchgebogenen Stellung bewegbar ist, in der der verformbare Abschnitt (51) zur Kolbenstange (3) hin mit der Durchbiegung (δ) zwischen 5% und 25% der Länge (L₁) der Kolbenstange (3) gebogen ist;
- sich die Schwächungsbereiche (510) entlang zueinander paralleler Achsen (A) erstrecken, wenn sich der verformbare Abschnitt (51) in der Ruhestellung befindet.

6. Spritze nach einem der Ansprüche 2 bis 5, wobei das erste Material starr ist und der starre Abschnitt (50) des manuellen Drückers (5) aus dem ersten Material gefertigt ist.

7. Spritze nach einem der Ansprüche 2 bis 5, wobei das erste Material flexibel ist.

8. Spritze nach Anspruch 1, wobei der verformbare Abschnitt (51) des manuellen Drückers (5) aus einem flexiblen Material mit einem Young'schen Modul gefertigt ist, der dazu geeignet ist, das flexible Material zur Kolbenstange (3) hin mit der Durchbiegung (δ) zwischen 5% und 25% der Länge (L₁) der Kolbenstange (3) zu biegen, wenn der Injektionsdruck manuell auf den manuellen Drücker (5) ausgeübt wird.

9. Spritze nach einem der Ansprüche 2 bis 7, wobei:
- der verformbare Abschnitt (51) des manuellen Drückers (5) zwischen einer Ruhestellung und einer durchgebogenen Stellung bewegbar ist, in der der verformbare Abschnitt (51) zur Kolbenstange (3) hin mit der Durchbiegung (δ) zwischen 5% und 25% der Länge (L₁) der Kolbenstange (3) gebogen ist;
- sich die Schwächungsbereiche (510) des ersten Materials jeweils entlang einer Achse (A) erstrecken, die senkrecht zu der ersten und der zweiten Längsachse (A₁, A₂) ist, wenn sich der verformbare Abschnitt (51) in der Ruhestellung befindet.

10. Spritze nach einem der Ansprüche 2 bis 7, wobei:
- der verformbare Abschnitt (51) des manuellen Drückers (5) zwischen einer Ruhestellung und einer durchgebogenen Stellung bewegbar ist, in der der verformbare Abschnitt (51) zur Kolbenstange (3) hin mit der Durchbiegung (δ) zwischen 5% und 25% der Länge (L₁) der Kolbenstange (3) gebogen ist;
- die Schwächungsbereiche (510) des ersten Materials einen Längsschnitt entlang der ersten Längsachse (A₁) aufweisen, der eine umgekehrte U-Form oder eine umgekehrte V-Form definiert, wenn sich der verformbare Abschnitt (51) in der Ruhestellung befindet und wenn die erste Längsachse (A₁) eine vertikale Achse ist, wobei sich das zweite Ende (31) der Kolbenstange (3) in Bezug auf die vertikale Achse oberhalb des ersten Endes (30) der Kolbenstange (3) befindet.

11. Spritze nach Anspruch 9 oder 10, wobei:
- der manuelle Drücker (5) entlang der zweiten Längsachse (A₂) eine Länge (L₂) aufweist;
- die Schwächungsbereiche (510) des ersten Materials entlang der zweiten Längsachse (A₂) eine Gesamtlänge (L₀) zwischen 30% und 50% der Länge (L₂) des manuellen Drückers (5) aufweisen, wenn sich der verformbare Abschnitt (51) in der Ruhestellung befindet.

12. Spritze nach einem der Ansprüche 1 bis 11, wobei:
- der manuelle Drücker (5) einen oberen Abschnitt, auf den ein zum Injizieren des Produkts geeigneter Injektionsdruck manuell ausgeübt wird, und einen dem oberen Abschnitt gegenüberliegenden unteren Abschnitt umfasst;
- der untere Abschnitt des manuellen Drückers (5) zwei Anschlagelemente (8) umfasst, die beiderseits der ersten Längsachse (A₁) angeordnet sind, um ein Verrutschen der Finger entlang der zweiten Längsachse (A₂) zu blockieren.

13. Spritze nach einem der Ansprüche 1 bis 12, wobei der manuelle Drücker (5) aus einem Kolbenkopf (310) besteht, der am zweiten Ende (31) der Kolbenstange (3) gebildet ist, wobei der Kolbenkopf (310) den starren Abschnitt (50) und den verformbaren Abschnitt (51) umfasst.

14. Spritze nach einem der Ansprüche 1 bis 12, wobei der manuelle Drücker (5) umfasst:
- einen Kolbenkopf (310), der starr ist und am zweiten Ende (31) der Kolbenstange (3) gebildet ist;
- ein Greifelement (7), das dazu angeordnet ist, den Kolbenkopf (310) zu bedecken.

15. Spritze nach Anspruch 14, wobei:
- das Greifelement (7) einen starren Bereich und einen verformbaren Bereich umfasst;
- der starre Abschnitt (50) des manuellen Drückers (5) durch den Kolbenkopf (310) und durch den starren Bereich des Greifelements (7) gebildet ist;
- der verformbare Abschnitt (51) des manuellen Drückers (5) durch den verformbaren Bereich des Greifelements (7) gebildet ist.

16. Spritze nach Anspruch 14, wobei:
- das Greifelement (7) einen verformbaren Bereich umfasst;
- der starre Abschnitt (50) des manuellen Drückers (5) durch den Kolbenkopf (310) gebildet ist;
- der verformbare Abschnitt (51) des manuellen Drückers (5) durch den verformbaren Bereich des Greifelements (7) gebildet ist.

17. Spritze nach einem der Ansprüche 1 bis 16, wobei die Länge (L₁) der Kolbenstange (3) zwischen 40 mm und 120 mm liegt.

## Claims

1. Manual syringe, comprising:
- a syringe body (1), intended to receive a product to be injected; the syringe body (1) having a first end (10), intended to be fitted with a needle holder, and an opposite second end (11);
- gripping flanges (2), arranged at the second end (11) of the syringe body (1);
- a piston rod (3), slidably mounted inside the syringe body (1), the piston rod (3) having opposite first and second ends (30, 31); the piston rod (3) having a length (L₁); the piston rod (3) extending along a first longitudinal axis (A₁);
- a stopper (4), arranged at the first end (30) of the piston rod (3);
- a manual pusher (5), arranged at the second end (31) of the piston rod (3), and on which an injection pressure suitable for injecting the product is manually applied; the manual pusher (5) extending along a second longitudinal axis (A₂), perpendicular to the first longitudinal axis (A₁); the manual pusher (5) comprising a rigid portion (50), formed by a central zone (Zc);
**characterized in that** the manual pusher (5) successively comprises a first lateral zone (ZL₁), the central zone (Zc) and a second lateral zone (ZL₂) along the second longitudinal axis (A₂); and **in that** the manual pusher (5) comprises a deformable portion (51), formed by the first lateral zone (ZL₁) and/or the second lateral zone (ZL₂), designed to flex toward the piston rod (3) by a deflection (δ) of between 5% and 25% of the length (L₁) of the piston rod (3), when the injection pressure is manually applied to the manual pusher (5).

2. Syringe according to claim 1, wherein the deformable portion (51) is made of a first material having a first Young's modulus; and the deformable portion (51) comprises weakening zones (510) of the first material, arranged according to the first Young's modulus to flex the first material toward the piston rod (3) by the deflection (δ) of between 5% and 25% of the length (L₁) of the piston rod (3), when the injection pressure is manually applied to the manual pusher (5).

3. Syringe according to claim 2, wherein each weakening zone (510) of the first material forms a material recess, the material recess preferably forming a groove within the first material.

4. Syringe according to claim 2 or 3, wherein the weakening zones (510) of the first material are arranged to form an array of periodic patterns.

5. Syringe according to any one of claims 2 to 4, wherein:
- the deformable portion (51) of the manual pusher (5) is movable between a rest position and a flexed position in which the deformable portion (51) is flexed toward the piston rod (3) by the deflection (δ) of between 5% and 25% of the length (L₁) of the piston rod (3);
- the weakening zones (510) extend along axes (A) parallel to each other when the deformable portion (51) is in the rest position.

6. Syringe according to any one of claims 2 to 5, wherein the first material is rigid, and the rigid portion (50) of the manual pusher (5) is made of the first material.

7. Syringe according to any one of claims 2 to 5, wherein the first material is flexible.

8. Syringe according to claim 1, wherein the deformable portion (51) of the manual pusher (5) is made of a flexible material having a Young's modulus suitable for flexing the flexible material toward the piston rod (3) by the deflection (δ) of between 5% and 25% of the length (L₁) of the piston rod (3), when the injection pressure is manually applied to the manual pusher (5).

9. Syringe according to any one of claims 2 to 7, wherein:
- the deformable portion (51) of the manual pusher (5) is movable between a rest position and a flexed position in which the deformable portion (51) is flexed toward the piston rod (3) by the deflection (δ) of between 5% and 25% of the length (L₁) of the piston rod (3);
- the weakening zones (510) of the first material each extend along an axis (A) perpendicular to the first and second longitudinal axes (A₁, A₂) when the deformable portion (51) is in the rest position.

10. Syringe according to any one of claims 2 to 7, wherein:
- the deformable portion (51) of the manual pusher (5) is movable between a rest position and a flexed position in which the deformable portion (51) is flexed toward the piston rod (3) by the deflection (δ) of between 5% and 25% of the length (L₁) of the piston rod (3);
- the weakening zones (510) of the first material have a longitudinal cross-section, along the first longitudinal axis (A₁), defining an inverted U-shape or an inverted V-shape when the deformable portion (51) is in the rest position and when the first longitudinal axis (A₁) is a vertical axis, the second end (31) of the piston rod (3) being located above the first end (30) of the piston rod (3) with respect to the vertical axis.

11. Syringe according to claim 9 or 10, wherein:
- the manual pusher (5) has a length (L₂) along the second longitudinal axis (A₂);
- the weakening zones (510) of the first material have a total length (L₀), along the second longitudinal axis (A₂), of between 30% and 50% of the length (L₂) of the manual pusher (5), when the deformable portion (51) is in the rest position.

12. Syringe according to any one of claims 1 to 11, wherein:
- the manual pusher (5) comprises an upper portion on which an injection pressure suitable for injecting the product is manually applied, and a lower portion opposite to the upper portion;
- the lower portion of the manual pusher (5) comprises two abutment members (8) arranged on either side of the first longitudinal axis (A₁) so as to block a sliding of fingers along the second longitudinal axis (A₂).

13. Syringe according to any one of claims 1 to 12, wherein the manual pusher (5) consists of a piston head (310) formed at the second end (31) of the piston rod (3), the piston head (310) comprising the rigid portion (50) and the deformable portion (51).

14. Syringe according to any one of claims 1 to 12, wherein the manual pusher (5) comprises:
- a piston head (310), rigid, formed at the second end (31) of the piston rod (3);
- a gripping member (7), arranged to cover the piston head (310).

15. Syringe according to claim 14, wherein:
- the gripping member (7) comprises a rigid zone and a deformable zone;
- the rigid portion (50) of the manual pusher (5) is formed by the piston head (310) and by the rigid zone of the gripping member (7);
- the deformable portion (51) of the manual pusher (5) is formed by the deformable zone of the gripping member (7).

16. Syringe according to claim 14, wherein:
- the gripping member (7) comprises a deformable zone;
- the rigid portion (50) of the manual pusher (5) is formed by the piston head (310);
- the deformable portion (51) of the manual pusher (5) is formed by the deformable zone of the gripping member (7).

17. Syringe according to any one of claims 1 to 16, wherein the length (L₁) of the piston rod (3) is between 40 mm and 120 mm.
